# EUROPEAN PATENT SPECIFICATION

(11) **EP 3 155 993 B1**
(45) Date of publication and mention of the grant of the patent: **29.08.2018**
(21) Application number: 16193336.1
(22) Date of filing: 11.10.2016
(51) Int. Cl.: A61B 18/00, A61B 18/14, A61M 25/00

(54) **ELECTROCATHETER WITH AN ELECTRODE COMPRISING A METAL BODY AND A PLASTIC COATING**
ELEKTROKATHETER MIT EINER EINEN METALLKÖRPER UND EINE KUNSTSTOFFBESCHICHTUNG UMFASSENDEN ELEKTRODE
ÉLECTROCATHÉTER AVEC UNE ÉLECTRODE COMPRENANT UN CORPS MÉTALLIQUE ET UN REVÊTEMENT EN PLASTIQUE

(30) Priority: 12.10.2015 IT UB20154603
(43) Date of publication of application: 19.04.2017
(73) Proprietor: Fiab S.P.A., 50039 Vicchio (IT)
(72) Inventor: FASANO, Antonio, 50139 FIRENZE (IT); CALABRO, Alberto, 50139 FIRENZE (IT)
(74) Representative: Paparo, Aldo

(56) References cited:
- US-A1- 2003 004 505
- US-A1- 2005 288 667
- US-A1- 2011 125 152
- US-A1- 2013 331 832

## Description

The present invention relates to an electrocatheter for surgical operations with at least one electrode and to a method for its manufacture.

It is known that in the treatment of certain disorders, such as atrial fibrillation in particular, use is made of techniques, known as ablation, which intervene preferably with radiofrequency irradiation in the vicinity of the outlet of the pulmonary veins in the left atrium and other cardiac regions, for the purpose to restore the correct electrical behavior of the affected tissues and prevent the emergence of the phenomenon of fibrillation.
In these techniques, use is therefore made of a radio-frequency electromagnetic field emitted by an ablation electrode, which produces an heating of tissue and of the
surrounding parts, which heating should be limited and kept under control. The propagation of heat in the surrounding tissue can cause lesions in various organs (phrenic nerve, the vagus nerve, the atrial wall, esophagus). Particularly dangerous are those concerning the esophagus (an atrio-esophageal fistula is an example of an almost always fatal injury). The temperature control in the esophageal lumen is possible via the insertion of a catheter with electrodes arranged along the catheter that are associated with thermal detectors.
These detectors are or comprise sensors that can be for example thermocouples or thermistors. For each electrode may be present also one or more detection conductors. Each of these conductors comprises at least one heat-sensitive element of a respective sensor, which is associated to a respective electrode. Such a heat-sensitive element is sensitive to the electrode temperature and / or the tissue in contact with or in proximity of the electrode itself. Each of these conductors is connected to the electrode by one part and by another part to the acquisition system for temperature detection.
The main body of the electrodes intended for heat detection is often made of metal. Such metal can interfere with the electromagnetic field emitted by the ablation generator (the antenna effect) giving rise to an undesired heating of the element itself with possible injuries induced in tissues in contact.
The document US 2003/004505 AA1 discloses an electrocatheter with an electrode including a metal body provided with a conductive coating. The object of the present invention is to develop a method of manufacturing an electrocatheter for surgical operations, which method allows to select, during the electrocatheter production phase, the material of the electrodes, in order to overcome the above concerns.
Additionally, the conductors that are connected to the electrodes are currently welded to the electrode material.
This determines the use of the welding material and the risk of damage or contamination to the electrodes and / or conductors.
Furthermore, the welding operation constitutes a further step of production, which determines an increase of the time required for the electrocatheter production.
Another object is to develop a method of manufacturing an electrocatheter for surgical operations that will decrease the production time compared to other methods.

A further object is to develop a method of manufacturing an electrocatheter for surgical operations that will decrease the risk of damage or contamination of the electrodes and / or conductors in the phase of electrocatheter production.
An electrocatheter for surgical is described, the electrocatheter comprising at least one catheter, at least one electrode and at least a conductor connected to said at least one electrode, said electrode being arranged on said catheter and said at least one electrode being made of at least partially with at least one plastic material. In this way one can select the at least one plastic material to adjust the electrical conductivity and / or the thermal conductivity of the electrode in an optimized way for the operations to be performed by the electrocatheter itself.
The electrocatheter can comprise at least one sensor for detecting the temperature of said at least one electrode and / or of a human's body tissue.
Said sensor may comprise for example a thermistor or a thermocouple. Such at least one conductor comprises at least one heat-sensitive element of said sensor.
Such at least one thermosensitive element can be integrated and / or incorporated in the electrode, at the time of formation of the latter.
In the case in which such a sensor is of the type with thermocouple, the electrocatheter comprises at least one further conductor in contact with said conductor and connected to said at least one electrode. Such at least one further conductor comprises at least one additional heat-sensitive element of said sensor.
Such at least one further heat-sensitive element can be integrated and / or incorporated in the electrode, at the time of formation of the latter. Preferably the electrocatheter comprises a plurality of electrodes. Preferably each of these electrodes is connected to a respective conductor and possibly to a respective additional conductor.
Each of these conductors may be suitable to transfer signals concerning of differences of electrical potentials or low-intensity current.
The present invention relates to a method of producing a electrocatheter for surgical operations, comprising a step of providing at least one electrode and at least one conductor, in which during this phase said at least one electrode is made partially with at least one plastic electrically Insulating material and at least a part of said at least one conductor is incorporated directly into the electrode body during the formation of said electrode.
Such a conductor that is incorporated in the electrode during the formation of the same, can be a conductor associated to a sensor or a conductor suitable for carrying current.
In case one wants to create a sensor of the thermocouple type, during said step is arranged at least one further conductor which is also connected to said electrode and of which at least part is incorporated directly into the electrode body during said formation, so that said conductors are integrated and / or embedded in the electrode and in contact with each other.
The at least one plastic material can be selected on the basis of the electrical and / or thermal conductivity desired. By adjusting the thermal conductivity at a high level, the electrocatheter can be used for instance in temperature measurements of various parts of living organisms without fear of altering the thermal inertia or interfering in temperature measurements during application of radio frequency energy. More generally, by adjusting the electrical conductivity at low levels, it is possible to minimize
the effects of magnetic fields, also variable, allowing the use of electrodes in security for example in NMR applications (Nuclear Magnetic Resonance) and / or RF (Radio Frequency).
The features of the present electrocatheter will be clarified in the following detailed description, offered by way of example and not limitative of the most general concepts claimed.
The following detailed description refers to the attached drawing, in which:
- In figure 1 a schematization is shown of a part of an electrocatheter with some components removed for clarity;
- In figure 2 a is shown of the electrocatheter of Figure 1.

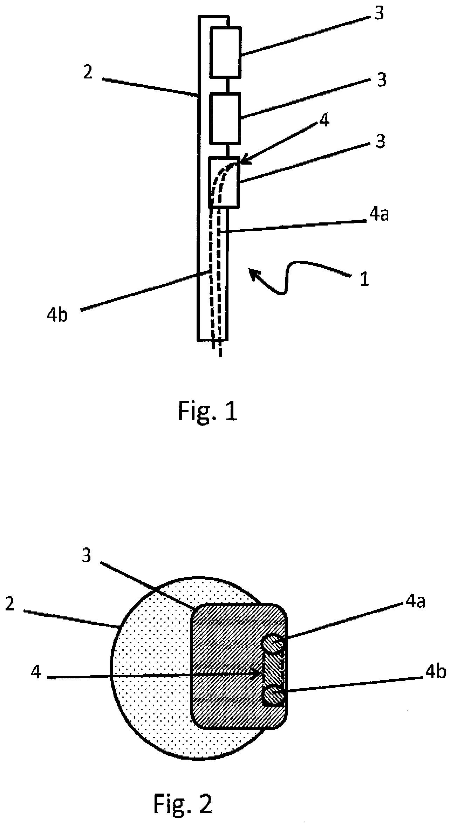

In figure 1 n electrocatheter 1 for surgical operations is shown. Such an electrocatheter 1 comprises at least one elongated body 2, preferably cylindrical, made of biocompatible material, such as silicone or polyurethane.

The electrocatheter 1 comprises a plurality of electrodes 3, and at least one conductor 4a, which can be defined as the first conductor 4a. The first conductor 4a is connected to said at least one electrode 3.
In the case shown, the electrocatheter 1 comprises at least one sensor 4 for detecting the temperature of at least one of said electrodes 3 and / or of a human body's tissue. Said first conductor 4a, in the case shown, performs the function of sensing element of the sensor 4, and thus comprises at least one heat-sensitive element which can be as the first heat-sensitive element. At least a portion of said first conductor 4a is integrated and / or embedded directly in the electrode 3.
This part of the first conductor 4a corresponds to this first heat-sensitive element, which is then integrated and / or incorporated directly in the electrode 3.
In another possible case, the first conductor 4a may be used as a current transfer to the respective electrode 3.
In the case shown, the sensor 4 may comprise for example a thermistor or a thermocouple.
In the case that sensor 4 is a thermocouple, the electrocatheter 1 comprises at least one further conductor 4b, which can be as the second conductor 4b.
This second conductor 4b, in case the sensor is a thermocouple 5, is in contact with the first conductor 4a, preferably in correspondence of a junction.

The second conductor 4b, in case the sensor is a thermocouple, is also connected to said at least one electrode 3.
The second conductor 4b, in case the sensor is a thermocouple, comprises at least one further element of said heat-sensitive sensor 4, which can be defined as a heat-sensitive element.
At least a part of the second conductor 4b is integrated and / or embedded directly in the electrode 3.
This part of the second conductor 4b corresponds to this second thermosensitive element, which is then integrated and / or incorporated directly in the electrode 3.
Note that, in Figure 1, the electrocatheter part 1 is shown greatly simplified and schematic. The catheter 2 is shown only in part, and the conductors 4a and 4b connected to one of the electrodes 3 are only partially shown.
Figure 2 shows a electrocatheter 1 section transverse to the axis of the catheter 2, and in correspondence a cross section of one of the electrodes 3. The electrodes 3 are distributed along the axis of the catheter 2. Figure 2 shows cross sections of the respective conductors 4a and 4b. It is noted that the first conductor 4a and the second conductor 4b are at least partially embedded in the electrode 3, and in contact with each other along the dotted portion that connects these sections.
These conductors 4a and 4b may be, for example, electric wires or electric cables. For example, in case the sensor 4 is a thermocouple, the conductors 4a and 4b could be made of copper and constantan.
The dashed portion of Figure 2 shows the sensor portion 4 that realizes the junction between the two conductors 4a and 4b.
Said portion is dashed because the cross section is considered a portion where the conductors 4a and 4b are not in contact.

The sensor 4 may however be of another type than those described. The present invention concerns a method of production of a electrocatheter 1 for surgical operations, comprising a step of providing at least one electrode 3 and at least a conductor 4a connected to said electrode 3. During this phase, said at least one electrode 3 is made partially with at least one plastic electrically conductive material. During this phase, at least a part of said at least one conductor 4a is incorporated directly into the electrode body 3 during the formation of said electrode 3.
Said at least a part of conductor 4a is preferably a terminal part. Depending on the chosen shape for the electrode, its production can be by molding, forming, injection or another suitable technique to create objects made in at least one plastic material.
To obtain the electrocatheter 1 referred to the attached figures, at least one further conductor 4b is used. This additional conductor 4b is to be connected to the electrode 3. In particular, at least a part of the further conductor 4b is incorporated directly into the electrode body 3 during electrode formation itself, so that said conductors 4a and 4b are integrated and / or embedded in the electrode 3 and in contact with each other. Said at least one part of the further conductor 4b is preferably a terminal part.
Said at least one of plastic material of electrode 3 can be selected on the basis of the electrical and\or thermal conductivity. After this stage, the electrode 3 together with that part of the conductor 4a and / or that part of the further conductor 4b, is positioned on the catheter 2 so as to remain fixed at the catheter 2. More precisely, by way of example only, some of particular interest may also be the following. Another possible case provides that the electrode includes a body and a coating located on at least part of the outer surface of said body. This body is made of at least one metal material, and the body may be completely metallic. Such a coating is realized by at least one plastic material, and may be fully realized by said at least one plastic material.
Such a coating can be biocompatible and has a very low electrical conductivity, but sufficiently high thermal conductivity. The combination of these features ensures protection of the metallic body without affecting the speed of response of the device to thermal signals.
The application of the coating can take place by immersion, coating, spray or electrostatic deposition. The body is prepared and one subsequently proceeds to the application or formation of such a coating by painting, spray or electrostatic deposition. In a different case, alternative to the application of the coating by paint or spray, it is possible to proceed to an appropriate chemical or electrochemical treatment of the outer surface of the body.
In this case the formation of the coating takes place by chemical or electrochemical treatment on at least part of the outer surface of the body. The treatment can be performed so as to produce an electromagnetic shielding with very low thickness, so as not to degrade the thermal transmission property. An example of such a treatment can be galvanic oxidation, as with anodized aluminum. Such a chemical or electrochemical treatment of the external surface of the body is suited to create an electrically insulating layer so thin as not to compromise the speed of electrode response to temperature changes. The electrode according to the invention is composite, and comprises a light metal element, which supports the welding to a thermal sensor, and which is immersed or mounted on a main body made of a plastic electrically Insulating material.

## Claims

1. Electrocatheter (1) for surgical operations, comprising at least one catheter (2), at least one electrode (3) and at least one conductor (4a) connected to said at least one electrode (3), said electrode (3) being disposed on said catheter (2), **characterized in that** said at least one electrode (3) is composite and comprises a metal element immersed in or mounted on a main body of a plastic electrically Insulating material.

2. Electrocatheter according to claim 1, wherein at least a part of said at least one conductor is built into and / or incorporated in said at least one electrode.

3. Electrocatheter according to claim 1 or 2, comprising at least one sensor (4) for the detection of the temperature of said at least one electrode and / or of a tissue of a human body, said at least one conductor (4a) comprising at least one heat-sensitive element of said sensor.

4. Electrocatheter according to claim 3. wherein said sensor Is a thermistor.

5. Electrocatheter according to claim 3, comprising at least one further conductor (4b) in contact with said conductor (4a) and connected to said at least one electrode (3), said at least one further conductor (4b) comprising at least one additional heat-sensitive element of said sensor (4), and wherein at least a part of said at least one additional conductor is built into and / or Incorporated In said at least one electrode.

6. Electrocatheter according to claim 5, wherein said sensor is a thermocouple.

7. Electrocatheter according to claim 1 or 2, wherein said at least one conductor is designed to transfer current to said at least one electrode.

8. Electrocatheter according to one or more of the preceding claims, comprising a plurality of electrodes.

9. A method for the production of a electrocatheter for surgical operations, comprising a step of providing at least one electrode and at least a conductor connected to said electrode, wherein during this step said at least one electrode is made partially from a metal element **characterized in that** the metal element is immersed in or mounted on a main body of a plastic electrically Insulating material.

10. Method according to claim 9, wherein at least a part of said at least one conductor is incorporated directly into the electrode body during the formation of said electrode.

11. Method according to claim 9 or 10, wherein during said step is arranged at least one additional conductor connected to said electrode and of which at least part is Incorporated directly into the electrode body during the formation, so that said conductors are at least partially built Into and I ur incorporated in the electrode and in contact with each other.

## Patentansprüche

1. Elektrokatheter (1) für chirurgische Eingriffe, umfassend mindestens einen Katheter (2), mindestens eine Elektrode (3) und mindestens einen Leiter (4a), der mit der mindestens einen Elektrode (3) verbunden ist, wobei die Elektrode (3) auf dem Katheter (2) angeordnet ist, **dadurch gekennzeichnet, dass** die mindestens eine Elektrode (3) zusammengesetzt ist und ein Metallelement umfasst, das in einen Hauptkörper aus einem elektrisch isolierenden Kunststoffmaterial eingetaucht oder darauf montiert ist.

2. Elektrokatheter nach Anspruch 1, wobei mindestens ein Teil des mindestens einen Leiters in die mindestens eine Elektrode eingebaut und/oder in diese aufgenommen ist.

3. Elektrokatheter nach Anspruch 1 oder 2, umfassend mindestens einen Sensor (4) zur Erfassung der Temperatur der mindestens einen Elektrode und/oder eines Gewebes eines menschlichen Körpers, wobei der mindestens eine Leiter (4a) mindestens ein wärmeempfindliches Element des Sensors umfasst.

4. Elektrokatheter nach Anspruch 3, wobei der Sensor ein Thermistor ist.

5. Elektrokatheter nach Anspruch 3, umfassend mindestens einen weiteren Leiter (4b) in Kontakt mit dem Leiter (4a) und verbunden mit der mindestens einen Elektrode (3), wobei der mindestens eine weitere Leiter (4b) mindestens ein zusätzliches wärmeempfindliches Element des Sensors (4) umfasst, und wobei mindestens ein Teil des mindestens einen zusätzlichen Leiters in die mindestens eine Elektrode eingebaut und/oder in diese aufgenommen ist.

6. Elektrokatheter nach Anspruch 5, wobei der Sensor ein Thermoelement ist.

7. Elektrokatheter nach Anspruch 1 oder 2, wobei der mindestens eine Leiter dazu ausgelegt ist, Strom auf die mindestens eine Elektrode zu übertragen.

8. Elektrokatheter nach einem oder mehreren der vorhergehenden Ansprüche, umfassend eine Vielzahl von Elektroden.

9. Verfahren zur Herstellung eines Elektrokatheters für chirurgische Eingriffe, umfassend einen Schritt des Bereitstellens mindestens einer Elektrode und mindestens eines mit der Elektrode verbundenen Leiters, wobei während dieses Schrittes die mindestens eine Elektrode teilweise aus einem Metallelement hergestellt wird, **dadurch gekennzeichnet, dass** das Metallelement in einen Hauptkörper aus einem elektrisch isolierenden Kunststoffmaterial eingetaucht oder darauf montiert wird.

10. Verfahren nach Anspruch 9, wobei mindestens ein Teil des mindestens einen Leiters während der Bildung der Elektrode direkt in den Elektrodenkörper aufgenommen ist.

11. Verfahren nach Anspruch 9 oder 10, wobei während des Schrittes mindestens ein zusätzlicher Leiter angeordnet ist, der mit der Elektrode verbunden ist und von dem mindestens ein Teil während der Bildung direkt in den Elektrodenkörper aufgenommen wird, so dass die Leiter mindestens teilweise in die Elektrode eingebaut und/oder in diese aufgenommen werden und miteinander in Kontakt stehen.

## Revendications

1. Électrocathéter (1) pour opérations chirurgicales, comprenant au moins un cathéter (2), au moins une électrode (3) et au moins un conducteur (4a) relié à ladite au moins une électrode (3), ladite électrode (3) étant disposée sur ledit cathéter (2), **caractérisé en ce que** ladite au moins une électrode (3) est composite et comprend un élément métallique intégré au ou monté sur un corps principal constitué d'un matériau plastique électriquement isolant.

2. Électrocathéter selon la revendication 1, dans lequel au moins une partie dudit au moins un conducteur est construite et/ou incorporée dans ladite au moins une électrode.

3. Électrocathéter selon la revendication 1 ou 2, comprenant au moins un capteur (4) pour la détection de la température de ladite au moins une électrode et/ou d'un tissu d'un corps humain, ledit au moins un conducteur (4a) comprenant au moins un élément thermosensible dudit capteur.

4. Électrocathéter selon la revendication 3, dans lequel ledit capteur est un thermistor.

5. Électrocathéter selon la revendication 3, comprenant au moins un conducteur (4b) supplémentaire en contact avec ledit conducteur (4a) et relié à ladite au moins une électrode (3), ledit au moins un conducteur (4b) supplémentaire comprenant au moins un élément thermosensible supplémentaire dudit capteur (4), et dans lequel au moins une partie dudit au moins un conducteur supplémentaire est construite et/ou incorporée dans ladite électrode.

6. Électrocathéter selon la revendication 5, dans lequel ledit capteur est un thermocouple.

7. Électrocathéter selon la revendication 1 ou 2, dans lequel ledit au moins un conducteur est conçu pour transférer du courant à ladite au moins une électrode.

8. Électrocathéter selon l'une ou plusieurs des revendications précédentes, comprenant une pluralité d'électrodes.

9. Procédé de fabrication d'un électrocathéter pour des opérations chirurgicales, comprenant une étape consistant à prévoir au moins une électrode et au moins un conducteur relié à ladite électrode, dans lequel durant cette étape ladite au moins une électrode est constituée partiellement d'un élément métallique, **caractérisé en ce que** l'élément métallique est intégré dans / ou monté sur un corps principal constitué d'un matériau plastique électriquement isolant.

10. Procédé selon la revendication 9, dans lequel au moins un conducteur est incorporé directement dans le corps de l'électrode pendant la formation de ladite électrode.

11. Procédé selon la revendication 9 ou 10, dans lequel pendant ladite étape un conducteur supplémentaire est au moins disposé relié à ladite électrode et duquel au moins une partie est incorporée directement dans le corps de l'électrode pendant la formation, de sorte que lesdits conducteurs sont au moins partiellement construits et/ou incorporés dans l'électrode et en contact réciproquement.
